# EUROPÄISCHE PATENTSCHRIFT

(11) **EP 2 353 013 B1**
(45) Veröffentlichungstag und Bekanntmachung des Hinweises auf die Patenterteilung: **14.09.2016**
(21) Anmeldenummer: 09748285.5
(22) Anmeldetag: 22.10.2009
(51) Int. Cl.: G01N 33/68, C12N 9/90

(54) **IMMUNOLOGISCHER TEST ZUM NACHWEIS VON AUTOANTIKÖRPERN GEGEN TESTIKULÄRE ANTIGENE**
IMMUNOLOGICAL TEST FOR DETECTING AUTOANTIBODIES AGAINST TESTICULAR ANTIGENS
TEST IMMUNOLOGIQUE POUR DETECTER DES AUTOANTICORPS POUR DES ANTIGENES TESTICULAIRES

(30) Priorität: 28.10.2008 DE 102008053503
(43) Veröffentlichungstag der Anmeldung: 10.08.2011
(73) Patentinhaber: Justus-Liebig-Universität Gießen, 35390 Gießen (DE)
(72) Erfinder: MEINHARDT, Andreas, 35037 Marburg (DE); FIJAK, Monika, 35037 Marburg (DE)
(74) Vertreter: Patentanwälte Olbricht Buchhold Keulertz
(86) Internationale Anmeldenummer: PCT/EP2009/063878
(87) Internationale Veröffentlichungsnummer: WO 2010/049340

(56) Entgegenhaltungen:
- WO-A2-2006/046108
- BOHRING C ET AL: "Isolation and identification of sperm membrane antigens recognized by antisperm antibodies, and their possible role in immunological infertility disease." MOLECULAR HUMAN REPRODUCTION FEB 2001, Bd. 7, Nr. 2, Februar 2001 (2001-02), Seiten 113-118, XP002564628 ISSN: 1360-9947
- MATHUR SUBBI P: "Autoimmunity in endometriosis: Relevance to infertility" AMERICAN JOURNAL OF REPRODUCTIVE IMMUNOLOGY, Bd. 44, Nr. 2, August 2000 (2000-08), Seiten 89-95, XP002564625 ISSN: 1046-7408
- FIJAK MONIKA ET AL: "Identification of immunodominant autoantigens in rat autoimmune orchitis" JOURNAL OF PATHOLOGY, Bd. 207, Nr. 2, Oktober 2005 (2005-10), Seiten 127-138, XP002564626 ISSN: 0022-3417
- ZHANG JINGJING ET AL: "ERp57 is a potential biomarker for human fertilization capability" MOLECULAR HUMAN REPRODUCTION, Bd. 13, Nr. 9, September 2007 (2007-09), Seiten 633-639, XP002564627 ISSN: 1360-9947

## Beschreibung

### Immunologischer Test zum Nachweis von Autoantikörpern gegen testikuläre Antigene

Die vorliegende Erfindung betrifft einen immunologischen Test zum Nachweis von Autoantikörpern gegen testikuläre Antigene, die als sehr relevant bei Formen immunologisch bedingter und infektionsbedingter Infertilität männlicher Säuger identifiziert wurden.

Der Test wird zur Diagnose und Therapiekontrolle von Erkrankungen verwendet, die mit einer erhöhten Menge an Autoantikörpern gegen testikuläre Antigene einhergehen, wie entzündungsbedingte männliche Fertilitätsstörungen wie z.B. still, aber auch der symptomatisch verlaufende Entzündungen in Hoden und in den begleitenden männlichen Geschlechtsorganen.

### Stand der Technik

Etwa jedes siebte Paar ist in Deutschland von ungewollter Kinderlosigkeit betroffen, wobei die Ursachen je rund zur Hälfte bei Mann und Frau zu finden sind. Auf Seiten des Mannes werden neben idiopathischer Infertilität (ca. 30%) urogenitale Infekte oder andere immunologische Faktoren (insgesamt ca. 12-15%) zu den wichtigsten Ursachen einer eingeschränkten Fertilität gerechnet. Sowohl systemische akute und chronische entzündliche Erkrankungen als auch lokale Infektionen und Entzündungen des männlichen Genitaltraktes spielen alleine oder als begleitende Ursache männlicher Fertilitätsstörungen stets eine wichtige Rolle. Chronische inflammatorische Veränderungen im Hoden (Hodenentzündung oder Orchitis) können zum Spermatogenese-Arrest und zu erheblichen Änderungen in der Spermatozoenzahl und -qualität führen. Die Nebenhodenentzündung (Epididymitis), deren Häufigkeit z.B. in den USA auf mehr als 600.000 Fälle pro Jahr geschätzt wird, tritt oft kombiniert als Epididymo-Orchitis auf. Klinisch bedeutsam bei Fertilitätsstörungen sind auch spermien-immobilisierende und/oder -agglutinierende Autoantikörper. Die Beurteilung der Relevanz immunologisch bedingter männlicher Infertilität und ihrer Folgen wird insbesondere dadurch erschwert, daß mit einer hohen Rate asymptomatischer Verläufe zu rechnen ist.

Die Diagnostik von Infektionen in den Samenwegen ist unbefriedigend. Sie stützt sich auf den Nachweis von Erregern, ein vermehrtes Auftreten von Leukozyten und/oder Entzündungsmediatoren im Ejakulat sowie eine verminderte Sekretionsleistung der akzessorischen Drüsen. Asymptomatische entzündliche Testesschäden sind in der Regel nur durch eine Hodenbiopsie sicher diagnostizierbar und bleiben dementsprechend als Ursache oder Kofaktoren von Fertilitätsstörungen häufig unerkannt. Folglich ist es denkbar, dass Entzündungen auch einen Teil des großen Patientenkollektivs mit idiopathischer Infertilität ausmachen und entzündliche Ursachen eine noch größere Bedeutung haben als bisher angenommen.

Deshalb besteht der Bedarf an einem Test, der geeignet ist, in einer Körperflüssigkeit oder biologischen Probe zur Identifizierung von entzündungsbedingten männlichen Fertilitätsstörungen wie z.B. still, aber auch symptomatisch verlaufende Entzündungen im Hoden und begleitenden männlichen Geschlechtsorganen eingesetzt zu werden, so dass die Durchführung einer Hodenbiopsie vermieden werden kann. Granulozyten Elastase und IL-6 sind leider nicht spezifisch und eignen sich daher nicht als Marker.

Gemäß einer Veröffentlichung von C. Bohring et al. in Molecular Human Reproduction, Vol. 7, No. 2, Seiten 113-118, 2001 (Isolation and identification of sperm membrane antigens recognized by antisperm antibodies, and thier possible role in immunological infertility disease) werden testikuläre Antikörper (ASA, antisperm antibodies), u.a. gegen das Antigen Disulfid Isomerase ER60, im Zusammenhang mit immunologischer Infertilität beschrieben.

Nach S. Mathur, American Journal of Reproductive Immunology, (Autoimmunity in Endometriosis: Relevance to Infertility), 2000, 44: 89-95, beschreibt endometrische, d.h. weibliche Autoantikörper gegen Transferrin, welche eine Rolle in der Endometriose-assoziierten Infertilität spielen. Männliche Infertilität wird nicht untersucht.

Eine Veröffentlichung von Fijak et al., Journal of Pathology, 2005, 207: 127-138, (Identification of immunodominant autoantigens in rat autoimmune orchitis), beschreibt, dass die Entzündung des Genitaltrakts ein Hauptgrund für die Infertilität des Mannes ist. Außerdem zeigt diese Veröffentlichung Experimente an Ratten, die durchgeführt wurden, um Moleküle zu identifizieren, die für eine Autoimmunattacke auf den Hoden verantwortlich sind. Dabei wurde ER60 als eine von insgesamt sechs Verbindungen gefunden. Die Detektion von Autoantikörpern mit Hilfe von testikulären Antigenen ist darin jedoch nicht beschrieben.

In Zhang et al., Molecular Human Reproduction, 2007, 13: 9, 633-639 (ERp57 is a potential biomarker for human fertiliozation capability) wird beschrieben, dass ERp57 (ER60)-Antikörper humane Spermien daran hindern, in zona-freie Hamsteroozyten zu penetrieren. Autoantikörper werden hierbei jedoch nicht erwähnt.

Die WO 2006/046108 A2 beschreibt eine Methode zur Diagnose von Fertilitätsstörungen eines männlichen Säugers, die auf eine geringe Samenqualität zurückzuführen sind. Bei diesem Diagnoseverfahren wird der Level von TFPI (Tissue Factor Pathway Inhibitor) gemessen. Dieser korreliert mit mehreren qualitätsrelevanten Parametern wie z.B. der Spermienanzahl oder der Spermienmotilität, weshalb durch die Bestimmung des TFPI-Levels eine Aussage über den Fertilitätszustand des Patienten getroffen werden kann. Das in WO 2006/046108 A2 beschriebene Verfahren weist verschiedene Nachteile auf. Zum einen liefert die Bestimmung des TFPI-Levels nur bedingt aussagekräftige Werte und zum anderen liefert die Untersuchungsmethode keinen klaren Hinweis auf die tatsächliche Ursache der Fertilitätsstörung. Eine Methode zur direkten Detektion von Autoantikörpern gegen testikuläre Antigene wird nicht offenbart.

Bislang ist noch kein geeigneter Marker bekannt, der zum spezifischen Nachweis auf entzündungsbedingte männliche Fertilitätsstörungen geeignet ist und in einem Test eingesetzt werden kann.

### Aufgabe

Aufgabe der vorliegenden Erfindung ist es, ein einfach durchzuführendes und nicht invasives Verfahren an einer biologischen Probe zum spezifischen Nachweis und damit für eine zuverlässige Diagnose von entzündungsbedingten männlichen Fertilitätsstörungen bereitzustellen. Weiterhin ist es die Aufgabe, einen Kit zur Durchführung des Verfahrens bereitzustellen.

### Lösung der Aufgabe

Diese Aufgabe wird erfindungsgemäß gelöst durch einen immunologischen Test zum Nachweis und zur spezifischen Bestimmung von Autoantikörpern gegen testikuläre Antigene, die für entzündungsbedingte männliche Fertilitätsstörungen spezifisch sind, in einer biologischen Probe gemäß der Ansprüche 1 oder 2.

Überraschenderweise wurden in eigenen Versuchen in biologischen Proben spezielle Autoantikörper gegen testikuläre Antigene als sehr relevant bei Formen immunologisch bedingter Infertilität des Mannes identifiziert.

Diese Ergebnisse zeigen, dass biologische Proben wie z.B. Seren von Patienten mit verschiedenen Formen testikulärer Entzündungen sehr häufig hohe ER-60-Antikörpertiter und Transferrin-Antikörpertiter aufweisen und sich damit von gesunden Probanden oder Männern mit nicht entzündlichen Spermatogeneseschäden (z. B. Sertoli cell only-Syndrom, Arrest der Spermatogenese, Oligo-Astheno-Teratozoospermie) abgrenzen lassen.

Dazu wurden Hodenextrakte von Patienten mit normaler Spermatogenese mittels dem Fachmann bekanntem 2D-SDS-PAGE aufgetrennt und auf Nitrozellulosemembranen transferiert, wie z.B. in Journal of Pathology (2005; 207, 127-138) offenbart. Die Blotmembranen wurden mit Kontrollseren von Frauen, von gesunden Probanden oder mit Serumproben von Patienten inkubiert, bei denen verschiedene Formen fokaler oder - seltener - voll entwickelter Hodenentzündungen nach andrologischer Untersuchung diagnostiziert worden waren. Autoreaktive Spots wurden mit Massenspektrometrie (MALDI-MS) untersucht und Proteine identifiziert, gegen die in Seren von Männern mit entzündlich bedingter Infertilität häufig Autoantikörper gerichtet waren:

| **Positive Seren/Gesamtzahl der Seren (% Reaktivität)** | **Identifiziertes Protein** |
|---|---|
| 12/13 (92%) | Disulfid-Isomerase ER-60 (Syn. Erp60, ERp57) |
| 8/13 (61 %) | Transferrin (Siderophilin) |

Die Kontrollseren zeigten mit keinem der identifizierten Proteinspots eine Reaktion.

Überraschenderweise zeigen die Proteine Disulfid-Isomerase ER-60 (Synonyme: ERp57, p58, Entry name: PDIA3_HUMAN) mit 92 % bzw. Transferrin (Siderophilin) mit 61 % eine stark spezifische Autoreaktivität mit Seren von Patienten mit unterschiedlichen Formen testikulärer Entzündung.

Damit wurden die Proteine Disulfid-Isomerase ER-60 (Synonyme: ERp57, p58, Entry name: PDIA3_HUMAN) und Transferrin (Siderophilin) als spezifische Marker für testikuläre Entzündungen männlicher Säuger identifiziert.

Unter Verwendung von Nukleinsäuresequenz und/oder Aminosäuresequenzen dieser Markerproteine wird ein immunologischer Test bereitgestellt, mit dem in biologischen Proben die Autoantikörper gegen die testikulären Antigene ER-60-Autoantikörper und/oder Transferrin-Autoantikörper, die mit entzündungsbedingten männlichen Fertilitätsstörungen einhergehen, spezifisch erfasst werden.

Der immunologische Test zum Nachweis und zur spezifischen Bestimmung von Autoantikörpern gegen testikuläre Antigene, die mit entzündungsbedingten männlichen Fertilitätsstörungen einhergehen, in einer biologischen Probe umfasst die Bindung der Autoantikörper gegen testikuläre Antigene, nämlich der ER-60-Autoantikörper und/oder Transferrin-Autoantikörper an das spezifische testikuläre Antigen ER-60 oder Transferrin.

Der erfindungsgemäße Test wird zur Diagnostik und Therapiekontrolle der still, aber auch der symptomatisch verlaufenden Entzündungen in Hoden und begleitenden Geschlechtsorganen männlicher Säuger verwendet.

Dazu wird in biologischen Proben männlicher Säuger die Menge an Autoantikörpern gegen die testikulären Antigene, nämlich der ER-60 Autoantikörper und/oder der Transferrin-Autoantikörper, ermittelt, die bei Patienten mit verschiedenen Formen testikulärer Entzündungen sehr häufig vorkommen. Der ermittelte Gehalt an Autoantikörpern gegen testikuläre Antigene wird dann mit einem Referenzwert verglichen.

In einer bevorzugten Ausführungsform der Erfindung umfasst der erfindungsgemäße Test die Ermittlung des Gehaltes an ER-60 Autoantikörpern in biologischen Proben, insbesondere Serum. Dazu wird als Marker das testikuläre Antigen, Protein ER-60, rekombinant bereitgestellt. Dies erfolgt ausgehend von der Nukleinsäuresequenz des ER-60 Proteins wie sie in Seq ID Nr.1 angegeben ist, mit Hilfe allgemein bekannter molekularbiologischer Methoden rekombinant z.B. durch Expression in E. coli und wie im Stand der Technik üblich, z.B. mittels HPLC Aufreinigung.

Erfindungsgemäß sind neben der in Seq ID Nr.1 offenbarten Nukleinsäuresequenz des Proteins Disulfid-Isomerase ER-60 (Synonyme: ERp57, p58, Entry name: PDIA3_HUMAN) auch Proteine umfasst, die durch geringfügige Änderungen der Nukleinsäuresequenz durch Additionen, Deletionen und/oder Substitutionen entstanden sind, sofern die Funktion des translatierten Proteins bei der Bindung von ER-60 Autoantikörpern nicht wesentlich beeinflusst wird.

Erfindungsgemäß sind neben der offenbarten Nukleinsäuresequenz des Proteins Disulfid-Isomerase ER-60 auch synonyme und homologe Proteine umfasst, wie z.B.: ERp57, p58, Entry name: PDIA3_HUMAN.

Eine biologische Probe ist jedes Material eines Säugetieres, insbesondere eines Menschen, das Autoantikörper beinhaltet, z.B. Körperflüssigkeit, wie Vollblut, Serum oder Plasma, Seminalplasma, Spinalflüssigkeit, peritoneale Flüssigkeit, Speichel, Tränenflüssigkeit oder Urin, außerdem Biopsiematerial oder Gewebe.

Die in der biologischen Probe befindlichen Autoantikörper gegen testikuläre Antigene, insbesondere die ER-60-Autoantikörper und/oder Transferrin-Autoantikörper werden durch spezifische Bindung an das jeweilige testikuläre Antigen, insbesondere ER-60 Protein bzw. Transferrin und anschließende Detektion mit geeigneten Nachweisverfahren erfasst.

Erfindungsgemäß erfolgt die Bestimmung der Autoantikörper gegen testikuläre Antigene, also die Bestimmung der ER-60-Autoantikörper und/oder Transferrin-Autoantikörper, mittels immunologischer Methoden. Insbesondere immuncytochemische Verfahren, die Bestimmung durch Radioimmunoassay (RIA) oder die Bestimmung durch einen "Enzyme linked Immunosorbent Assay" (ELISA) haben sich als besonders geeignet erwiesen. Alternativ ist der Test auf einem Testträger mit möglichst geringem Volumenbedarf, z.B. auf einem Teststreifen, angeordnet.

Die Durchführung des immunologischen Tests umfasst das Inkontaktbringen einer Blutprobe oder einer anderen biologischen Probe eines männlichen Säugers, deren Gehalt an Autoantikörpern gegen testikuläre Antigene bestimmt werden soll, mit adsorbiertem spezifischem testikulärem Antigen, mindestens eine Waschung, um die nichtadsorbierten Bestandteile zu beseitigen und den Nachweis der an das adsorbierte Antigen gebundenen Autoantikörper durch eine Zubereitung von IgG-Antikörpern, die spezies-spezifisch für den zu untersuchenden männlichen Säuger sind und spezies-spezifisch an den Autoantikörper binden können, z.B. im Falle eines Menschen, eine Zubereitung von anti-human IgG-Antikörpern, die so markiert sind, dass sie durch eine Kaskade von Reaktionen des Typs Biotin-Streptavidin- Peroxidase oder -Alkalische Phosphatase nachgewiesen werden.

In einer bevorzugten Ausführung erfolgt die Durchführung indem das zu bindende Markerprotein, insbesondere das testikuläre Antigen, Protein ER-60 oder Transferrin, an einer Oberfläche beispielsweise einer Mikrotiterplatte, einer Polyvinylchloridplatte oder einer geeigneten Gewebeplatte oder einem Teststreifen immobilisiert wird und unspezifische Bindungen abgesättigt werden. Anschließend wird die zu untersuchende biologische Probe möglichst in flüssiger Form hinzugefügt, wobei darin enthaltener Autoantikörper an das testikuläre Antigen bindet, ER-60 Autoantikörper bindet an das immobilisierte Protein ER-60 bzw. Transferrin Autoantikörper bindet an das immobilisierte Transferrin. Alternativ erfasst der Test auch ER-60 Autoantikörper und Transferrin Autoantikörper, wenn Protein ER-60 oder Transferrin an einer Oberfläche immobilisiert sind. Nach mindestens einem Waschschritt wird ein den Autoantikörper gegen testikuläre Antigene bindender Sekundärantikörper hinzugefügt, der mit einem Enzym, beispielsweise einer alkalischen Phosphatase oder Peroxidase, gekoppelt ist. Nach mindestens einem erneuten Waschschritt wird der Probe schließlich ein farbloses bzw. nichtfluoreszierendes Substrat für das am zweiten Antikörper gebundene Enzym zugefügt. Dieses Enzym wandelt das Substrat zu einem farbigen oder fluoreszierenden Produkt um, dessen Konzentration durch einen geeigneten Detektor, beispielsweise ein Photometer, bestimmt werden kann.

Mit diesem immunologischen ELISA-Test lassen sich selbst geringe Mengen an Autoantikörpern gegen testikuläre Antigene, insbesondere die ER-60 Autoantikörper und/oder Transferrin Autoantikörper, schnell und zuverlässig nachweisen. Ein weiterer Vorteil dieses Tests besteht darin, dass er mit gebräuchlichen ELISA-Geräten und kommerziell erhältlichen Mikrotiterplatten im 96-, 256- oder gar 1024-Format betreibbar ist, weshalb sich eine große Anzahl von Analysen gleichzeitig durchführen lässt.

Bei dem RIA-Verfahren finden im Prinzip die gleichen Schritte wie beim ELISA-Verfahren statt, ausgenommen, dass anstelle eines mit einem geeigneten Enzym konjugierten zweiten Antikörpers ein radioaktiv markierter zweiter Antikörper eingesetzt wird. Die quantitative Detektion erfolgt in diesem Fall beispielsweise mittels eines Scintillationszählers.

Abgesehen von den vorgenannten Verfahren kann die Bestimmung der Autoantikörper gegen testikuläre Antigene durch Bindung an das Markerprotein, also die Bindung von ER-60 Autoantikörper an das immobilisierte Protein ER-60 bzw. die Bindung von Transferrin Autoantikörper an das immobilisierte Transferrin auch mit anderen dem Fachmann bekannten immunologischen Methoden qualitativ und quantitativ erfasst werden, wobei lediglich beispielsweise das "Western-Blot"-Verfahren bzw. das Dot-Blot-Verfahren genannt sei.

Alternativ ist das testikuläre Antigen, insbesondere Protein ER-60 und/oder Transferrin auf einem Testträger mit möglichst geringem Volumenbedarf, z.B. auf einem Teststreifen mit ein oder mehreren saugfähigen Matrices auf einem geeigneten Trägermaterial angebracht und wird dann mit der biologischen Probe, möglichst in flüssiger Form in Kontakt gebracht, so dass darin enthaltene Autoantikörper gegen testikuläre Antigene binden und über geeignete Nachweissysteme z.B. durch die Bildung einer sichtbaren Linie erfasst werden können.

Vorzugsweise erfolgt die qualitative oder quantitative Erfassung der Autoantikörper gegen testikuläre Antigene, insbesondere die Bindung von ER-60 Autoantikörper an das immobilisierte Protein ER-60 bzw. die Bindung von Transferrin Autoantikörper an das immobilisierte Transferrin aus Körperflüssigkeiten wie Serum oder Plasma eines Individuums, wobei als Wert für die Bindung die Fluoreszenz z.B. als optische Dichte (OD) bei einer Wellenlänge von 450 und 570 nm gemessen wird. Der Absorptionswert ergibt sich als Differenz zwischen der 450 nm und 570 nm Wellenlänge.

Dieser Wert wird mit einem Referenzwert verglichen. Dieser wird z.B. aus Messdaten von biologischen Proben gesunder männlicher Säugetiere und/oder männliche Säugetiere mit diagnostizierter Hodenentzündung ermittelt.

Alternativ wird eine Probe mit definierter Konzentration von testikulärem Antigen, insbesondere ER-60 oder Transferrin eingesetzt und der Referenzwert beispielsweise aus Vergleichskurven oder Vergleichstabellen ermittelt oder in Form von Vergleichswerten zugrunde gelegt.

Vorzugsweise bestimmt man den Referenzwert parallel zur Analyse der Probe. Doppelbestimmungen von Referenzwert und Probe sind alternativ möglich.

Beispielsweise wird der Referenzwert aus Messdaten von biologischen Proben gesunder Spender ermittelt, wobei der Referenzwert dann vorzugsweise unter einem OD-Wert von 0,35 liegt. Beispielsweise wird der Referenzwert aus Messdaten von biologischen Proben von Spendern mit diagnostizierter still oder symptomatisch verlaufender Hodenentzündung ermittelt, wobei der Referenzwert dann über einem OD-Wert 0,35, bevorzugt über 0,4 liegt.

Liegt der Gehalt an Autoantikörpern gegen testikuläre Antigene, insbesondere den ER-60 Autoantikörpern und/oder den Transferrin Autoantikörpern der biologischen Probe über einem OD-Wert 0,35, bevorzugt über 0,4, ist dies ein Hinweis auf Vorhandensein einer entzündungsbedingten Fertilitätsstörung.

Der immunologische Test ermöglicht eine verbesserte nicht-invasive Diagnosestellung, besonders bei biologischen Proben von Patienten mit still oder symptomatisch verlaufenden entzündungsbedingten Fertilitätsstörungen und vermeidet die Durchführung einer Hodenbiopsie.

Mit dem erfindungsgemäßen immunologischen Test werden die ER-60 Autoantikörper und/oder Transferrin Autoantikörper nachgewiesen. Damit ist es möglich, die Diagnose auf testikuläre Entzündungen versus entzündliche Spermatogeneseschäden (Sertoli cell only-Syndrom) abzugrenzen. Es handelt sich um eine neue systematische Analyse, bei der auch diskrete Veränderungen wie disseminiert und in geringer Dichte vorkommende Lymphozyten berücksichtigt werden. Der erfindungsgemäße immunologische Test wird auch verwendet, um den Erfolg einer Therapie einer entzündungsbedingten Fertilitätsstörung zu überprüfen.

Da sich der erfindungsgemäße immunologische Test einfach standardisieren lässt, kann er sowohl in der Humanmedizin, insbesondere bei der Überwachung von *in vitro*-Fertilisationen oder bei Fertilisationsdiagnosen und Fertilisationsgutachten, als auch im Rahmen der modernen Tierzucht eingesetzt werden.

Die biologische Probe stammt erfindungsgemäß aus einem männlichen Säuger, besonders bevorzugt aus einem Menschen.

Der erfindungsgemäße immunologische Test kann leicht in Form eines gebrauchsfertigen "Kits" dargeboten werden, welcher ER-60 und/oder Transferrin Antigen, das an eine Oberfläche eines Trägers adsorbiert ist, umfasst und eine Zubereitung von IgG-Antikörpern, die spezies-spezifisch für den zu untersuchenden männlichen Säuger sind und spezies-spezifisch an den Autoantikörper binden können, z.B. im Falle eines Menschen, eine Zubereitung von anti-human IgG-Antikörpern, die so markiert sind, dass sie durch eine Kaskade von Reaktionen des Typs Biotin-Streptavidin- Peroxidase oder -Alkalische Phosphatase nachgewiesen werden.

Alternativ umfasst der Kit einen Träger auch Puffer und Reagenzien z.B. Reagenzien, die für den Nachweis der Reaktion erforderlich sind wie z.B. Streptavidin, das mit einem Marker gekoppelt ist, der eine Farbreaktion ergibt.

Alternativ umfasst der Kit zusätzlich eine Standardprobe von ER-60 Antikörpern oder Transferrin-Antikörpern zum Eichen des Kits, wobei zum Nachweis von ER-60 Autoantikörpern eine Standardprobe von ER-60 Antikörpern eingesetzt wird und zum Nachweis von Transferrin Autoantikörpern eine Standardprobe von Transferrin-Antikörpern.

### Ausführungsbeispiele

### 1.Bereitstellung von ER-60

Bevorzugt wird rekombinantes, in E. coli exprimiertes Protein Disulphide Isomerase ER-60 (Synonyme: ERp57, p58, Entry name: PDIA3_HUMAN) bereitgestellt. Dies erfolgt beispielsweise aus einem Expressionsklon, der die DNA-Sequenz von humanem ER-60 trägt, z.B. pET-hER-60-Expressionsklon, wie z.B. beschrieben in Urade et al., J. Biochem. 122, 834-842; 1997. Das Protein wird exprimiert, z.B. in E. coli BL21 (DE3), beispielweise nach dem Protokoll von Urade et al. J Biol Chem. 267 (21):15152-15159) und aufgereinigt z.B. mittels HPLC.

Dazu wird das Expressionsplasmid pET-hER60 in *E.coli* BL21(DE3) transformiert und unter geeigneten Bedingungen kultiviert, z.B. in Anwesenheit von Ampicillin (500 µg/ml) in 400 ml Medium, z.B. LB-Medium (1% Trypton, 0,5% Hefeextrakt, 1% NaCl; pH 7.0) bei 30°C. Die Proteinexpression wird bei einem geeigneten OD₆₀₀ₙₘWert, z.B. bei OD₆₀₀ₙₘWert=0.5 induziert, z.B. mit 0.5 mM iso-propyl-1-thio-β-D-Galactopyranosid (IPTG) bei 30°C über ca. 2h. Anschließend wird die bakterielle Kultur geerntet und das Pellet unter geeigneten Bedingungen aufgenommen und präzipitiert, z.B. in 10ml Puffer, z.B. 20mM HEPES-KOH (pH 6.8), 50mM KCl, 5mM EDTA (pH 8.0), 1mM PMSF resuspendiert und mit Ultraschall behandelt. Das Zelllysat wird ca. 30 min bei 4°C bei 5000 x g abzentrifugiert und der Überstand mit 60 und 70% saturiertem Ammoniumsulfat 30 min bei 4°C präzipitiert und anschließend bei 5000 x g für 30 min abzentrifugiert. Das Proteinpellet wird in 500µl Puffer z.B. 20mM HEPES-KOH (pH 6.8), 50mM KCl, 5mM EDTA (pH 8.0), 1mM DTT resuspendiert und gereinigt, z.B. mittels Gelfiltration (Superdex 200 HR) in der HPLC. In einem alternativen Schritt werden die Fraktionen, die hER-60 beinhalten, zusätzlich aufgereinigt, z.B. mit einer Mono Q Säule z.B. in 10ml Puffer, z.B. 20mM HEPES-KOH (pH 6.8), 50mM KCl, 5mM EDTA (pH 8.0), 1mM DTT.

Die Proteinreinheit wird überprüft, z.B. in einem SDS-PAGE Gel.

### 2. Immunologischer Test

Eine ELISA-Platte z.B. MaxiSorp ELISA-Platte (Nunc) wird mit dem rekombinanten ER-60, z.B. 2,5 µg/ml über Nacht bei 4°C in 0,1 M Natriumcarbonat, pH 9.5 beschichtet, mit geeignetem Puffer gewaschen, z.B. Phosphatpuffer (PBS, pH 7.2 + 0,05% Tween-20) und geblockt, z.B. mit 2% Magermilchpulver in PBS bei Raumtemperatur (RT) 2,5 Stunden. Nach der Blockierung unspezifischer Bindungsstellen werden jeweils die Kontroll-Proben und die zu überprüfenden Serum-Proben in einer Verdünnungsreihe (von unverdünnt bis 1:1000 im Blocklösung) in Duplikaten auf die beschichtete Platte aufgetragen und 1 Stunde bei RT inkubiert. Nach der Inkubationszeit werden die Wells mindestens einmal gewaschen and anschließend mit einem zweitem sezies-spezifischen Antikörper, z.B. einem anti-human IgG-Biotin (1:100 000 in Blocklösung) mindestens eine Stunde bei RT inkubiert. Nach mindestens einem Waschschritt werden die Proben mit Streptavidin - Meerrettichperoxidase (GE Healthcare) 1:4000 im Waschpuffer für 20 min bei RT inkubiert. Nach mindestens einem Waschschritt wird die Farbreaktion z.B. mit TMB (3,3',5,5'-tetramethylbenzidine; BD Biosciences) für 20 min entwickelt. Die Farbreaktion wird gestoppt, z.B. mit 2N H₂SO₄. Die Absorption (OD-Wert) wird bei der Wellenlänge 450 und 570 nm gemessen. Der Absorptionswert ergibt sich als Differenz zwischen der 450 nm und 570 nm Wellenlänge.

### 3. Auswertung

Vorzugsweise erfolgt die Auswertung des immunologischen Tests durch Quantifizierung der ER-60 Autoantikörper in biologischen Proben, bevorzugt Serum oder Plasma. Der ermittelte Messwert wird mit einem Referenzwert verglichen. Dieser wird z.B. aus Messdaten von Proben gesunder Spender ermittelt, wobei er dann vorzugsweise unter einem OD-Wert 0,35 liegt. Alternativ wird der Referenzwert aus Messdaten von Proben von Spendern mit diagnostizierter Hodenentzündung ermittelt, wobei der Referenzwert dann über einem OD-Wert 0,35, bevorzugt über 0,4 liegt. Alternativ wird eine Probe mit definierter Konzentration von ER-60 eingesetzt und der Referenzwert beispielsweise aus Vergleichskurven oder Vergleichstabellen ermittelt oder in Form von Vergleichswerten zugrunde gelegt. Vorzugsweise bestimmt man den Referenzwert parallel zur Analyse der Probe.

Liegt der Gehalt an ER-60 Autoantikörpern der Probe über einem OD-Wert 0,35, bevorzugt über 0,4, ist dies ein Hinweis auf Vorhandensein einer entzündungsbedingten Fertilitätsstörung des männlichen Säugers.

### 4.Validierung

Zur Validierung des erfindungsgemäßen immunologischen Tests werden größere Versuchsserien durchgeführt, wobei der Nachweis der ER-60 Autoantikörper in einer Kontrollgruppe mit Seren von Menschen ohne andrologisch relevante Vorerkrankungen und normalen Ejakulatbefund mit verschiedenen Patientengruppen verglichen wird.

Die Patientengruppen setzen sich zusammen aus
1. Seren von Männern mit eingeschränkter Fertilität. Einschlusskriterium ist eine Spermienkonzentration unter 10 Millionen/ml, bei denen keine Anhaltspunkte für Infektionen oder Entzündungen vorliegen.
2. Seren von Männern, die auf der Basis einer Ejakulatuntersuchung die Kriterien einer Samenwegsentzündung erfüllen.
3. Seren von Männern, bei denen durch eine Hodenbiopsie eine testikuläre Entzündung gefunden wurde.
4. Seren von Männern, deren erkannte testikuläre bzw. Samenwegsentzündung mit Diclofenac behandelt wurde, um zu prüfen, ob die ER-60 Autoantikörpertiter mit einem Behandlungserfolg korrelieren.

## Patentansprüche

1. Verfahren zum Nachweis von Fertilitätsstörungen männlicher Säugetiere in einer biologischen Probe eines männlichen Säugers, **dadurch gekennzeichnet, dass** es sich um ein immunologisches Verfahren zur spezifischen Bestimmung von Autoantikörpern gegen testikuläre Antigene, die mit entzündungsbedingten Fertilitätsstörungen einhergehen handelt, wobei das Verfahren die Bindung der Autoantikörper an das spezifische testikuläre Antigen umfasst, und wobei als Autoantikörper gegen testikuläre Antigene spezifisch ER-60 Autoantikörper oder Transferrin Autoantikörper bestimmt werden.

2. Verfahren zum Nachweis von Fertilitätsstörungen männlicher Säugetiere in einer biologischen Probe eines männlichen Säugers, **dadurch gekennzeichnet, dass** es sich um ein immunologisches Verfahren zur spezifischen Bestimmung von Autoantikörpern gegen testikuläre Antigene, die mit entzündungsbedingten Fertilitätsstörungen einhergehen handelt, wobei das Verfahren die Bindung der Autoantikörper an das spezifische testikuläre Antigen umfasst, und wobei als Autoantikörper gegen testikuläre Antigene spezifisch ER-60 Autoantikörper und Transferrin Autoantikörper bestimmt werden.

3. Immunologisches Verfahren gemäß Anspruch 1, **dadurch gekennzeichnet, dass** das spezifische testikuläre Antigen ER-60 oder Transferrin ist.

4. Immunologisches Verfahren gemäß Anspruch 1, **dadurch gekennzeichnet, dass** das spezifische testikuläre Antigen ER-60 und Transferrin ist.

5. Immunologisches Verfahren gemäß der vorangegangenen Ansprüche, **dadurch gekennzeichnet, dass** der Nachweis der Autoantikörper gegen testikuläre Antigene immuncytochemisch, durch Radioimmunoassay (RIA) oder durch Enzyme-linked-Immunosorbent Assay (ELISA) erfolgt.

6. Immunologisches Verfahren gemäß der vorangegangenen Ansprüche, **dadurch gekennzeichnet, dass** der Nachweis der Autoantikörper gegen testikuläre Antigene auf einem Teststreifen mit geringem Volumenbedarf erfolgt.

7. Immunologisches Verfahren gemäß der vorangegangenen Ansprüche, **dadurch gekennzeichnet, dass** der ermittelte Gehalt an Autoantikörpern gegen testikuläre Antigene mit einem Referenzwert verglichen wird.

8. Immunologisches Verfahren gemäß der vorangegangenen Ansprüche, **dadurch gekennzeichnet, dass** seine Durchführung umfasst:
- das Inkontaktbringen einer Blutprobe oder einer anderen biologischen Probe eines männlichen Säugers, deren Gehalt an Autoantikörpern gegen testikuläre Antigene bestimmt werden soll, mit adsorbiertem spezifischem testikulärem Antigen
- mindestens eine Waschung, um die nichtadsorbierten Bestandteile zu beseitigen
- und den Nachweis der an das adsorbierte Antigen gebundenen Autoantikörper gegen testikuläre Antigene durch eine Zubereitung von IgG-Antikörpern, die spezies-spezifisch für den zu untersuchenden männlichen Säuger sind und spezies-spezifisch an den Autoantikörper gegen testikuläre Antigene binden können und die so markiert sind, dass sie durch eine Kaskade von Reaktionen des Typs Biotin-Streptavidin-Peroxidase oder - Alkalische Phosphatase nachgewiesen werden.

9. Verwendung des immunologischen Verfahrens gemäß der vorangegangenen Ansprüche zur Messung des Gehaltes an Autoantikörpern gegen testikuläre Antigene, die mit entzündungsbedingten Fertilitätsstörungen männlicher Säugetiere einhergehen, in einer biologischen Probe zur Bestimmung auf das Vorhandensein immunologisch bedingter und infektionsbedingter Infertilität bei männlichen Säugetieren.

10. Kit zur Durchführung des Verfahrens nach einem der vorangegangenen Ansprüche, **dadurch gekennzeichnet, dass** er umfasst:
- spezifisches testikuläres Antigen, das an einen Träger adsorbiert ist, wobei das spezifische testikuläre Antigen ER-60 und/oder Transferrin ist,
- und eine Zubereitung von IgG-Antikörpern, die spezies-spezifisch für den zu untersuchenden männlichen Säuger sind und spezies-spezifisch an den Autoantikörper gegen testikuläre Antigene binden können und die so markiert sind, dass sie durch eine Kaskade von Reaktionen des Typs Biotin-Streptavidin-Peroxidase oder -Alkalische Phosphatase nachgewiesen werden.

## Claims

1. Procedure for the detection of fertility disorders of male mammals in a biological sample from a male mammal, **characterized in that** the method is an immunological procedure for the specific determination of autoantibodies against testicular antigens, which are associated with inflammation-related fertility disorders, wherein the method comprises binding of the autoantibodies to the specific testicular antigen, and wherein specifically ER-60 autoantibodies or transferrin autoantibodies are determined as autoantibodies against testicular antigens.

2. Procedure for the detection of fertility disorders of male mammals in a biological sample from a male mammal, **characterized in that** the method is an immunological test for the specific determination of autoantibodies against testicular antigens which are associated with inflammation-related fertility disorders, wherein the method comprises binding of the autoantibodies to the specific testicular antigen, and wherein specifically ER-60 autoantibodies and transferrin autoantibodies are determined as autoantibodies against testicular antigens.

3. Immunological procedure according to claim 1, **characterized in that** the specific testicular antigen is ER-60 or transferrin.

4. Immunological procedure according to claim 1, **characterized in that** the specific testicular antigen is ER-60 and transferrin.

5. Immunological procedure according to any of the preceding claims, **characterized in that** the detection of autoantibodies against testicular antigens is performed immunocytochemically, by radioimmunoassay (RIA) or by enzyme-linked-immunosorbent assay (ELISA).

6. Immunological procedure according to any of the preceding claims, **characterized in that** the detection of autoantibodies against testicular antigens is performed on a test strip with low volume requirement.

7. Immunological procedure according to any of the preceding claims, **characterized in that** the detected value of autoantibodies against testicular antigens is compared to a reference value.

8. Immunological procedure according to any of the preceding claims, **characterized in that** performing the procedure comprises:
- bringing a blood sample or other biological specimen from a male mammal whose content of autoantibodies against testicular antigens is to be determined into contact with adsorbed specific testicular antigen;
- washing at least once to remove non-adsorbed components; and
- detecting autoantibodies against testicular antigens bound to the adsorbed antigen by a preparation of IgG-antibodies, the IgG-antibodies being species-specific for the male mammal to be investigated and able to bind species-specifically to the autoantibodies against testicular antigens, and being labelled such that they can be detected in a cascade of reactions of the biotin-streptavidin-peroxidase or alkaline phosphatase type.

9. Use of the immunological procedure according to any of the preceding claims for measurement of the concentration of autoantibodies against testicular antigens, which are associated with inflammation-related fertility disorders of male mammals, in a biological sample for detecting a presence of immunological-related and infection-related fertility disorders of male mammals.

10. Kit for carrying out a procedure according to any of the preceding claims, **characterized by** comprising
- specific testicular antigen adsorbed to a carrier, wherein the specific testicular antigen is ER-60 and/or transferrin, and
- a preparation of IgG-antibodies, the IgG-antibodies being species-specific for the male mammal to be investigated and able to bind species-specifically to the autoantibody against testicular antigens, and being labelled such that they can be detected in a cascade of reactions of the biotin-streptavidin-peroxidase or alkaline phosphatase type.

## Revendications

1. Procédé pour la détection de troubles de la fécondité de mammifères mâles dans un échantillon biologique d'un mammifère mâle, **caractérisé en ce qu'**il s'agit d'un procédé immunologique pour la détermination spécifique d'auto-anticorps contre des antigènes testiculaires, qui s'accompagnent de troubles de la fécondité dus à une inflammation, dans lequel le procédé comprend la liaison des auto-anticorps à l'antigène testiculaire spécifique, et dans lequel spécifiquement un auto-anticorps ER-60 ou un auto-anticorps de la transferrine sont déterminés comme auto-anticorps contre des antigènes testiculaires.

2. Procédé pour la détection de troubles de la fécondité de mammifères mâles dans un échantillon biologique d'un mammifère mâle, **caractérisé en ce qu'**il s'agit d'un procédé immunologique pour la détermination spécifique d'auto-anticorps contre des antigènes testiculaires, qui s'accompagnent de troubles de la fécondité dus à une inflammation, dans lequel le procédé comprend la liaison des auto-anticorps à l'antigène testiculaire spécifique, et dans lequel spécifiquement un auto-anticorps ER-60 et un auto-anticorps de la transferrine sont déterminés comme auto-anticorps contre des antigènes testiculaires.

3. Procédé immunologique selon la revendication 1, **caractérisé en ce que** l'antigène testiculaire spécifique est l'ER-60 ou la transferrine.

4. Procédé immunologique selon la revendication 1, **caractérisé en ce que** l'antigène testiculaire spécifique est l'ER-60 et la transferrine.

5. Procédé immunologique selon les revendications précédentes, **caractérisé en ce que** la détection des auto-anticorps contre des antigènes testiculaires s'effectue de façon immunocytochimique, par essai radio-immunologique (RIA) ou par essai immuno-enzymatique (ELISA).

6. Procédé immunologique selon les revendications précédentes, **caractérisé en ce que** la détection des auto-anticorps contre des antigènes testiculaires s'effectue sur des bandes de test nécessitant un faible volume.

7. Procédé immunologique selon les revendications précédentes, **caractérisé en ce que** la teneur déterminée en auto-anticorps contre des antigènes testiculaires est comparée à une valeur de référence.

8. Procédé immunologique selon les revendications précédentes, **caractérisé en ce que** sa réalisation comprend :
- la mise en contact d'un échantillon de sang ou d'un autre échantillon biologique d'un mammifère mâle, dont la teneur en auto-anticorps contre des antigènes testiculaires doit être déterminée, avec un antigène testiculaire adsorbant spécifique
- au moins un lavage pour éliminer les composants non adsorbants
- et la détection des auto-anticorps contre des antigènes testiculaires liés à l'antigène adsorbé par une préparation d'anticorps IgG qui sont spécifiques à une espèce pour le mammifère mâle à analyser et qui peuvent se lier de façon spécifique à une espèce à l'auto-anticorps contre des antigènes testiculaires et qui sont marqués de telle sorte qu'ils sont détectés par une cascade de réactions du type biotine-streptavidine-peroxydase ou phosphatase alcaline.

9. Utilisation du procédé immunologique selon les revendications précédentes pour la mesure de la teneur en auto-anticorps contre des antigènes testiculaires, qui s'accompagnent de troubles de la fécondité dus à une inflammation chez des mammifères mâles, dans un échantillon biologique pour la détermination de la présence d'une stérilité d'origine immunologique et due à une infection chez des mammifères mâles.

10. Kit pour la réalisation du procédé selon l'une des revendications précédentes, **caractérisé en ce qu'**il comprend :
- un antigène testiculaire spécifique qui est adsorbé sur un support, dans lequel l'antigène testiculaire spécifique est l'ER-60 et/ou la transferrine,
- et une préparation d'anticorps IgG qui sont spécifiques à une espèce pour le mammifère mâle à analyser et qui peuvent se lier de façon spécifique à une espèce à l'auto-anticorps contre des antigènes testiculaires et qui sont marqués de telle sorte qu'ils sont détectés par une cascade de réactions du type biotine-streptavidine-peroxydase ou phosphatase alcaline.
